# EUROPEAN PATENT APPLICATION

(11) **EP 0 743 046 A1**
(43) Date of publication of application: **20.11.1996**
(21) Application number: 96201335.5
(22) Date of filing: 15.05.1996
(51) Int. Cl.: A61F 2/01, A61B 17/22

(54) **Filter catheter**

(30) Priority: 19.05.1995 NL 1000400; 12.10.1995 NL 1001410
(71) Applicant: CORDIS EUROPA N.V., NL-9301 LJ Roden (NL)
(72) Inventor: Homsma, Tjeerd, 9302 ER Roden (NL); Boudewijn, Alexander Christiaan, 9351 KS Leek (NL)
(74) Representative: 't Jong, Bastiaan Jacobus

(57) **Abstract**

The invention relates to a catheter which comprises at least one tubular basic body with a proximal and a distal end. Herein arranged on the distal end is a filter element which is formed by a number of flexible strips which are distributed over the periphery and extend in longitudinal direction of the basic body. These strips are each curved substantially arcuately in outward direction and can be pressed together elastically.

## Description

The invention relates to a catheter comprising in per se known manner at least one tubular basic body and a proximal and a distal end.

In medical operations wherein there is a danger of blood thrombi spreading through the vascular system of a patient, filters are placed in the bloodstream which catch these thrombi.

The present invention relates to a catheter which is provided on its distal end with such a filter element. The filter element is formed by a number of flexible strips which are distributed over the periphery and extend in longitudinal direction of the basic body and which are each curved substantially arcuately in outward direction and which can be pressed together elastically. The catheter is carried with the filter element in the pressed together state to the correct location in the bloodstream of the patient, whereafter the strips are elastically released to a position close to or in contact with the wall of the blood vessel. The blood can flow through between the strips but possible thrombi are held back. Infusion wires or catheters with which the thrombi can be broken down can be introduced through the central lumen.

A suitable embodiment is characterized in claim 2. The material of the catheter is of a nature herein such that a deformation arranged during heating to a temperature close to the softening temperature is retained after cooling. The arcuate curve in the strips can thus be arranged.

In preference the step of claim 3 is applied herein. The flexible strips hereby acquire a better movement potential and can thus still place themselves reliably close to or in contact with the wall of the blood vessel, also in difficult conditions.

A further development is characterized in claim 4. When the filter element is in its active position the relatively distal ends of the strips are spread, so that the strips define to a certain extent a funnel shape which leads to the lumen of the basic body.

A further favourable development is characterized in claim 5. The inner tubular body provided with the filter element is introduced into the patient in the retracted position together with the outer tubular body. As soon as the outer tubular body is positioned in the correct manner, the inner tubular body can be placed into the extended position in which the filter is active.

A suitable embodiment is characterized herein in claim 6. The inner tubular body can hereby slide easily in the outer body.

According to a further development the step of claim 7 is applied. When the filter element is placed into the extended position the hemostasis device is adjusted whereby the sealed passage is pinched securely round the inner tubular body. The relative position of the inner tubular body relative to the outer tubular body in the extended active position is hereby fixed so that the inner tubular body cannot move unintentionally into the retracted position.

By applying the step of claim 8 a flushing liquid can be supplied to a position close to the filter element via the remaining diameter of the lumen.

With the step of claim 9 is achieved that at the proximal end the relative position of the distal ends of the inner and outer tubular body can be accurately monitored, and particularly whether the filter element is placed in correct manner in the active extended position or in the protective retracted position.

In order to prevent the filter element adhering to the tissue of the blood vessel after a period of time, the step of claim 10 is preferably applied. Filter catheters of the present type can thus remain for a long time in the body of the patient.

The invention will be further elucidated in the following description with reference to the annexed figures.

Figure 1 shows schematically the application of the catheter according to the invention.

Figure 2 shows a partly broken away and schematic view of an embodiment of a catheter according to the invention during insertion into a patient.

Figure 3 shows a view corresponding with figure 2 of the catheter in the active situation.

Figure 4 shows a partly broken away view of the end of a preferred embodiment of the catheter in the situation corresponding with figure 2.

Figure 5 shows the end of the catheter of figure 4 in the situation corresponding with figure 3.

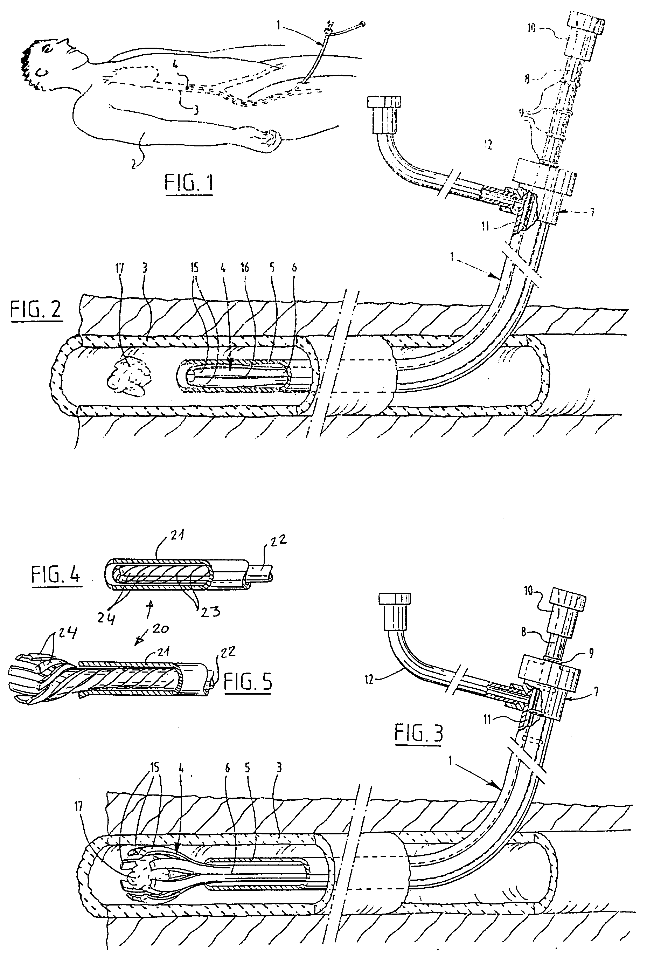

Fig. 1 shows schematically that a catheter 1 according to an embodiment of the invention is introduced into a blood vessel 3 of a patient 2. The distal end of catheter 1 bears a filter element 4 to be further described which can catch blood thrombi so that these do not reach locations in the blood circulation where this could be dangerous for the patient.

As shown more particularly in fig. 2, the catheter 1 comprises a tubular basic body built up of an outer tubular body 5 with a lumen 11 in which is received an inner tubular body 6.

At the proximal end, that is, the end of catheter 1 situated outside the patient during use, a hemostasis device 7 is arranged on the outer tubular body 5. This device further comprises in per se known manner a sealed passage through which the proximal end 8 of the inner tubular body 6 can extend to the outside.

On the distal end the inner tubular body 6 is provided with a number of flexible strips 15 extending in longitudinal direction of the basic body. In the embodiment shown these strips 15 are formed by a number of axial incisions 16 in the tubular body 6. The strips 15 are preformed such that in released state they are curved arcuately outward as shown in fig. 3. This released form can be arranged in the strips by heating them to a temperature close to the softening temperature of the material of the basic body, forming the strips into the desired shape and subsequently cooling them. The formed shape is then "frozen" into the material. The strips 15 can be elastically pressed together to substantially the diameter of the inner tubular body 6, so that filter element 4 formed by the arcuate strips 15 can be received into the outer tubular body 5.

In this situation as shown in fig. 2, the catheter is introduced into the patient. In the usual manner the end of the outer tubular body 5 takes an atraumatic form so that this can be carried out without possible trauma for the patient.

The proximal end 8 of the inner tubular body 6 is provided with a number of markings 9 which are shown here as thickenings but which can also be embodied in a contrasting colour or the like. The markings 8 co-act with the distal end of the outer tubular body 5, or more particularly the hemostasis device 7, in order to indicate the relative shifted position of the two tubular bodies.

When the catheter 1 is inserted, care is taken that the proximal end part 8 of the inner tubular body 6 remains a determined number of markings 9 outside the hemostasis device 7, thus ensuring that the filter element 4 remains in the retracted position.

When the distal end of the catheter is placed in the correct position, the filter element 4 can be shifted outward, while the outer tubular body 5 is simultaneously retracted, whereby the filter element 4 remains at the same position. This situation is shown in fig. 3. The proximal end part 8 is pressed inward a predetermined number of markings 9 into the hemostasis device 7 while the outer tubular body 5 is retracted over the same distance, so that the distal end part has moved outward in the same position from the distal end of tubular body 5.

The strips 15 are herein expanded into their "frozen" arcuate state so that they lie elastically against the wall of blood vessel 3. In this situation any thrombus 17 which may be carried along in the bloodstream is caught between the spread strips 15 so that it does not move any further.

The filter can be flushed regularly by supplying a flushing liquid into the lumen 11 of the outer tubular body 5 via a flushing connection 12 arranged for that purpose on the hemostasis device 7.

At least the strips 15 are coated on their outer side with a layer of bioactive material such as heparin so that, also in the case of a prolonged presence in the body, the wall of the blood vessel 3 and the filter element 4 are prevented from growing together.

After a period of time, when the danger of thrombosis has receded, the catheter can be removed again. To this end the inner tubular element is first retracted a predetermined number of markings 9 so that the filter element 4 is pressed together elastically and received into the distal end of the outer tubular body 5. The whole catheter can then be removed.

The hemostasis device 7 is preferably of an adjustable type whereof the sealed passage through which passes the proximal end 8 of the inner tubular body 6 can be pinched securely round the inner tubular body 6. The danger of the inner tubular body shifting in the outer body which certainly occurs during prolonged use is hereby avoided in reliable manner.

Fig. 4 and 5 show the distal end portion of a preferred embodiment. Just as catheter 1, the catheter 20 comprises an outer tubular body 21 in which an inner tubular body 22 is slidably received. In the end portion of the inner tubular body 22 are arranged cuts 23 which in this embodiment extend helically.

In the active situation shown in fig. 5 the strips 24 formed between the cuts 23 bend outward to form the filter element. Due to the helical form of strips 24 these are very flexible and can adapt well to the conditions, in particular they can place themselves very well against the wall of the blood vessel.

## Claims

1. Catheter comprising at least one tubular basic body with a proximal and a distal end, wherein on the distal end is arranged a filter element which is formed by a number of flexible strips which are distributed over the periphery and extend in longitudinal direction of the basic body and which are each curved substantially arcuately in outward direction and which can be pressed together elastically.

2. Catheter as claimed in claim 1, wherein the strips are formed by wall parts of the basic body defined between incisions of the basic body.

3. Catheter as claimed in claim 2, wherein the incisions extend helically.

4. Catheter as claimed in claim 2, wherein the strips are mutually separate on the distal outer end of the catheter.

5. Catheter as claimed in any of the foregoing claims comprising an outer tubular basic body and an inner tubular body which is received in a lumen thereof and which bears on its distal end the flexible strips and wherein the inner tubular body is slidable in the outer tubular body at least between a retracted position in which the strips are situated inside the outer tubular body and an extended position in which the flexible strips are situated outside the outer tubular body.

6. Catheter as claimed in claim 5, wherein on the proximal end of the outer tubular body is arranged a hemostasis device which comprises a sealed passage through which the proximal end of the inner tubular body extends to the outside.

7. Catheter as claimed in claim 6, wherein the hemostasis device is of the adjustable type, the sealed passage of which can be pinched securely round the inner tubular body.

8. Catheter as claimed in claim 7, wherein the hemostasis device is provided with a flushing connection connected to the lumen of the outer tubular body.

9. Catheter as claimed in any of the claims 5-8, wherein the proximal end of the inner tubular body is provided with a marking which co-acts with the proximal end of the outer tubular body and which shows the relative position of the distal ends of the outer and the inner tubular body.

10. Catheter as claimed in any of the foregoing claims, wherein the strips are provided with a coating of bioactive material such as heparin.
